# EUROPEAN PATENT SPECIFICATION

(11) **EP 2 114 885 B1**
(45) Date of publication and mention of the grant of the patent: **27.01.2016**
(21) Application number: 07750610.3
(22) Date of filing: 09.02.2007
(51) Int. Cl.: C07D 213/34, C07D 213/61, C07D 213/64, C07D 409/04

(54) **PROCESS FOR THE OXIDATION OF CERTAIN SUBSTITUTED SULFILIMINES TO INSECTICIDAL SULFOXIMINES**
VERFAHREN ZUR OXIDATION BESTIMMTER SUBSTITUIERTER SULFILIMINE ZU INSEKTIZIDEN SULFOXIMINEN
PROCÉDÉ D'OXYDATION DE CERTAINES SULFYLIMINES SUBSTITUÉES EN SULFOXIMINES INSECTICIDES

(43) Date of publication of application: 11.11.2009
(73) Proprietor: Dow AgroSciences LLC, Indianapolis, IN 46268-1054 (US)
(72) Inventor: ARNDT, Kim, E., Carmel, IN 46032 (US); BLAND, Douglas, C., Midland, MI 48642 (US); PODHOREZ, David, E., Midland, MI 48640 (US); MCCONNELL, James, R., Midland, MI 48640 (US)
(74) Representative: Weickmann & Weickmann
(86) International application number: PCT/US2007/003783
(87) International publication number: WO 2008/097235

(56) References cited:
- WO-A-2007/095229
- US-A1- 2005 228 027
- P. KIRSCH, ET AL.: "Synthesis and structural characterisation of highly fluorinated sulphimides and sulphoximides as functional building blocks for materials science" EUROPEAN JOURNAL OF ORGANIC CHEMISTRY, no. 9, May 2005 (2005-05), pages 797-802, XP002457170 WILEY-VCH VERLAG, WEINHEIM, DE
- R.S. GLASS, ET AL.: "Diels-Alder reactions of S-vinyl-S-arylsulphoximines" JOURNAL OF ORGANIC CHEMISTRY, vol. 49, no. 9, 4 May 1984 (1984-05-04), pages 1527-1533, XP002457171 AMERICAN CHEMICAL SOCIETY, WASHINGTON, DC, US
- J.E.G. KEMP, ET AL.: "Penicillin and cephalosporin sulphoximines" TETRAHEDRON LETTERS, vol. 20, no. 39, 1979, pages 3785-3788, XP002105510 ELSEVIER SCIENCE PUBLISHERS, AMSTERDAM, NL ISSN: 0040-4039

## Description

The present invention concerns a process for preparing insecticidal sulfoximines from certain substituted sulfilimines.

The substituted sulfilimines are useful intermediates for the preparation of certain new insecticides; see, for example, U.S. Patent Publication 2005/0228027. It would be advantageous to produce insecticidal sulfoximines efficiently and in high yield from the corresponding sulfilimines.

The present invention concerns a process for the oxidation of certain substituted sulfilimines, having the general structure of (I), 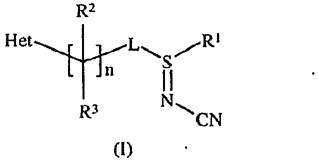 wherein
Het represents: 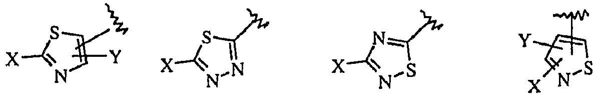 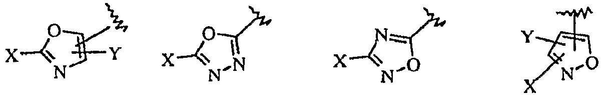 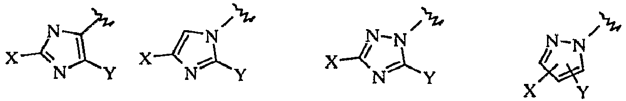 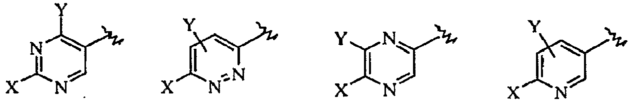
X represents halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, NO₂, SOₘR⁶ where m is an integer from 0-2, COOR⁴ or CONR⁴R⁵;
Y represents hydrogen, halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, NO₂, SOₘR¹ where m is an integer from 0-2, COOR⁴, CONR⁴R⁵, aryl or heteroaryl;
n is an integer from 0-3;
L represents either a single bond, -CH(CH₂)ₚ- where R¹, S and L taken together represent a 4-, 5-, or 6-membered ring and p is an integer from 1-3, -CH(CH₂OCH₂)- where R¹, S and L taken together represent a 6-membered ring, or -CH- where L, R² and the common carbon to which they connect taken together represent a 4-, 5-, or. 6-membered ring with up to, but no more than, 1 heteroatom.
R¹ represents C₁-C₄ alkyl, C₁-C₄ haloalkyl, C₃-C₆ alkenyl, C₃-C₆ alkynyl, C₃-C₆ haloalkenyl, arylalkyl, heteroarylalkyl, or -CH₂- in cases where R¹, S and L taken together represent a 4-, 5-, or 6-membered ring;
R² and R³ independently represent hydrogen, halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, SOₘR⁶ where m is an integer from 0-2, COOR⁴, CONR⁴R⁵, arylalkyl, heteroarylalkyl, or R² and R³ and the common carbon to which they attach form a 3-6 membered ring;
R⁴ and R⁵ independently represent hydrogen, C₁-C₄ alkyl, C₁-C₄ haloalkyl; C₃-C₆ alkenyl, C₃-C₆ alkynyl, C₃-C₆ haloalkenyl, aryl, heteroaryl, arylalkyl or heteroarylalkyl; and
R⁶ represents C₁-C₄ alkyl, C₁-C₄ haloalkyl, C₃-C₆ alkenyl, C₃-C₆ alkynyl, C₃-C₆ haloalkenyl, arylalkyl or heteroarylalkyl;
to form insecticidal sulfoximines having the structure (Ia): 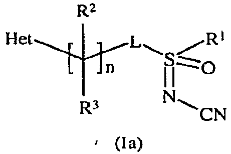 wherein
R¹, R², R³, Het, L and n are as previously defined. In the process, the sulfilimine of formula I is oxidized to the corresponding sulfoximine of formula Ia by contacting the sulfilimine in a suitable organic solvent that is essentially inert to the strong oxidizing conditions with an oxidizing agent comprising
an alkali metal permanganate at a temperature from -10 to 45°C.

The process is well suited to oxidize sulfilimines of the following classes:
(1) Compounds of formula (I) wherein Het is (6-substituted)pyridin-3-yl or (2-substituted)thiazol-5-yl and where X is halogen or C₁-C₂ haloalkyl and Y is hydrogen
(2) Compounds of formula (I) wherein R² and R³ are as previously defined, R¹ is methyl, n is 1, and L is a single bond, having the structure: 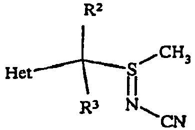
(3) Compounds of formula (I) wherein n is 1, R¹, S and L taken together form a standard 4-, 5-, or 6-membered ring such that L is -CH(CH₂)ₚ-and p is an integer from 1-3, and R¹ is -CH₂- having the structure: 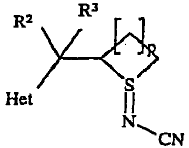
(4) Compounds of formula (I) wherein n is 0, R¹, S and L taken together form a standard 4-, 5-, or 6-membered ring such that L is -CH(CH₂)ₚ-and p is an integer from 1-3, and R¹ is -CH₂- having the structure:

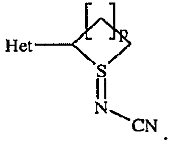

Throughout this document, all temperatures are given in degrees Celsius, and all percentages are weight percentages unless otherwise stated.

The terms "alkyl", "alkenyl" and "alkynyl", as well as derivative terms such as "alkoxy", "acyl", "alkylthio", "arylalkyl", "heteroarylalkyl" and "alkylsulfonyl", as used herein, include within their scope straight chain, branched chain and cyclic moieties. Thus, typical alkyl groups are methyl, ethyl, 1-methylethyl, propyl, 1,1-dimethylethyl, and cyclopropyl. Unless specifically stated otherwise, each may be unsubstituted or substituted with one or more substituents selected from but not limited to halogen, hydroxy, alkoxy, alkylthio, C₁-C₆ acyl, formyl, cyano, aryloxy or aryl, provided that the substituents are sterically compatible and the rules of chemical bonding and strain energy are satisfied. The term "haloalkyl" and "haloalkenyl" includes alkyl and alkenyl groups substituted with from one to the maximum possible number of halogen atoms, all combinations of halogens included. The term "halogen" or "halo" includes fluorine, chlorine, bromine and iodine, with fluorine being preferred. The terms "alkenyl" and "alkynyl" are intended to include one or more unsaturated bonds.

The term "aryl" refers to a phenyl, indanyl or naphthyl group. The term "heteroaryl" refers to a 5- or 6-membered aromatic ring containing one or more heteroatoms, viz., N, O or S; these heteroaromatic rings may be fused to other aromatic systems. The aryl or heteroaryl substituents may be unsubstituted or substituted with one or more substituents selected from halogen, hydroxy, nitro, cyano, aryloxy, formyl, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, C₁-C₆ acyl, C₁-C₆ alkylthio, C₁-C₆ alkylsulfinyl, C₁-C₆ alkylsulfonyl, aryl, C₁-C₆ OC(O)alkyl, C₁-C₆ NHC(O)alkyl, C(O)OH, C₁-C₆C(O)Oalkyl, C(O)NH₂, C₁-C₆ C(O)NHalkyl, or C₁-C₆C(O)N(alkyl)₂, provided that the substituents are sterically compatible and the rules of chemical bonding and strain energy are satisfied.

The sulfilimine starting materials of Formula I are the subject matter of a patent application filed concurrently with this application and certain of them have been disclosed in U.S. Patent Publication 2005/0228027. They can be prepared from the corresponding sulfides according to the following Schemes A and B.

The compounds of formula (I), wherein R¹, R², R³, n, and L are as previously defined can be prepared by the methods illustrated in Scheme A.

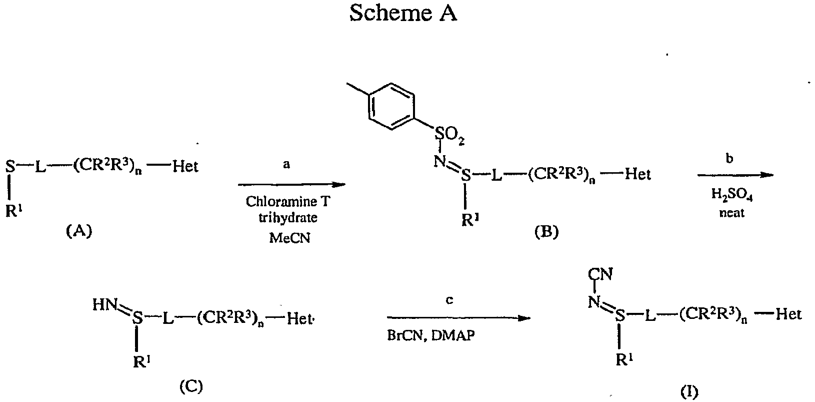

In step *a* of Scheme A, sulfide of formula (A) is iminated with chloramine T trihydrate a polar solvent at 25-60°C to provide an *N-*tosylsulfilimine of formula (B). In most cases, acetonitrile is the preferred solvent for the imination.

In step *b* of Scheme A, *N*-tosylsulfilimine (B) is hydrolyzed in neat sulfuric acid to provide the *N*-unsubstituted sulfilimine (C). This product is typically used directly in the next reaction without further purification.

In step c of Scheme A, the nitrogen of sulfilimine (C) can be cyanated with cyanogen bromide in the presence of a base to provide *N*-substituted sulfilimine (I).

The compounds of formula (Ia), wherein Het, R¹, R², R³, n, and L are as previously defined can be prepared by the method illustrated in Scheme B. Accordingly, the precursor sulfide is oxidized with iodobenzene diacetate in the presence of cyanamide at 0°C to give sulfilimine (Ia). The reaction can be carried out in a polar aprotic solvent like CH₂Cl₂.

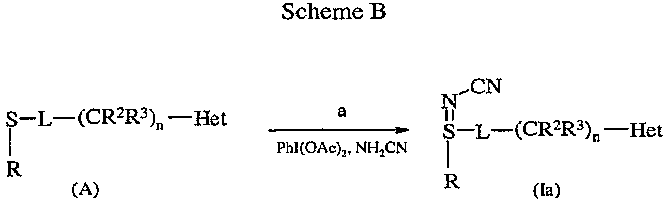

The precursor sulfides (A) can, in turn, be prepared in different ways as illustrated in Schemes C, D, E, F, G, H and I.

In Scheme C, the sulfide of formula (A₁), wherein L is a single bond, n is 1, R³ = H, and R¹, R² and Het are as previously defined can be prepared from halides of formula (D) by nucleophilic substitution with the sodium salt of an alkyl thiol.

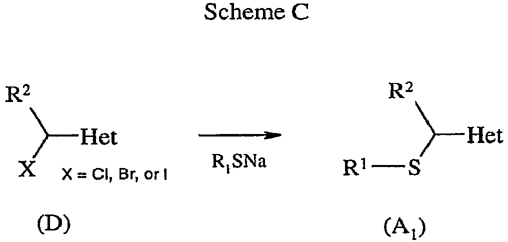

In Scheme D, the sulfide of formula (A₂), wherein L is a single bond, n is 3, R³ = H, and R¹, R² and Het are as previously defined, can be prepared from the chloride of formula (E) by reacting with a 2-mono substituted methyl malonate in the presence of base such as potassium *tert*-butoxide to provide 2,2-disubstitued malonate, hydrolysis under basic conditions to form a diacid, decarboxylation of the diacid by heating to give a monoacid, reduction of the monoacid with borane-tetrahyrofuran complex to provide an alcohol, tosylation of the alcohol with toluenesulfonyl chloride (tosyl chloride) in the presence of a base like pyridine to give a tosylate and replacement of the tosylate with the sodium salt of the desired thiol.

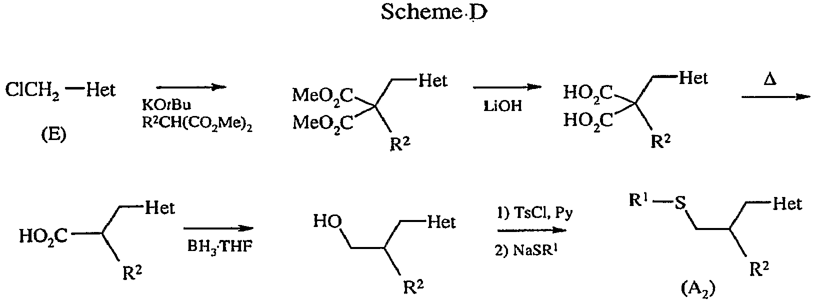

In Scheme E, the sulfide of formula (A₃), wherein L is a single bond, n is 2, R³ = H, and R¹, R² and Het are as previously defined, can be prepared from the nitrile of formula (F) by deprotonation with a strong base and alkylation with an alkyl iodide to give α-alkylated nitrile, hydrolysis of the α-alkylated nitrile in the presence of a strong acid like HCl to give an acid, reduction of the acid with borane-tetrahyrofuran complex to provide an alcohol, tosylation of the alcohol with tosyl chloride in the presence of a base like pyridine to give a tosylate and replacement of the tosylate with the sodium salt of the desired thiol.

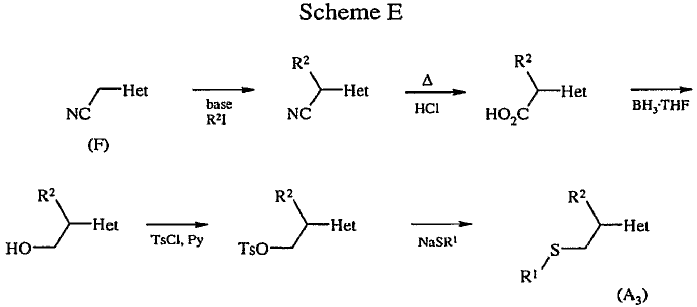

In Scheme F, the sulfide of formula (A₄), wherein n is 0, R¹ is -CH₂-, L is -CH(CH₂)ₚ- where p is either 2 or 3 and, taken together with R¹, S and L form a 5-or 6-membered ring, and Het is as previously described can be prepared from tetrahydrothiophene (p=2) or pentamethylene sulfide (p=3) (G). Chlorination of the cyclic sulfide starting material with *N*-chlorosuccinimide in benzene followed by alkylation with certain lithiated heterocycles or Grignard reagents can lead to the desired sulfides (A₄) in satisfactory yield.

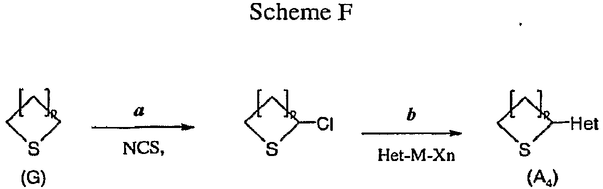

A more efficient protocol to access cyclic sulfides of formula (A₄) is illustrated in Scheme G where Het is a 6-substituted pyridin-3-yl and Z is previously defined. Accordingly, thiourea is added to a substituted chloromethyl pyridine, which, after hydrolysis, and alkylation with the appropriate bromo chloroalkane (p = 1, 2, or 3) under aqueous base conditions, yields sulfide (H). Subsequent cyclization of (G) in the presence of a base like potassium-*t*-butoxide in a polar aprotic solvent such as THF provides cyclic sulfide (A₄).

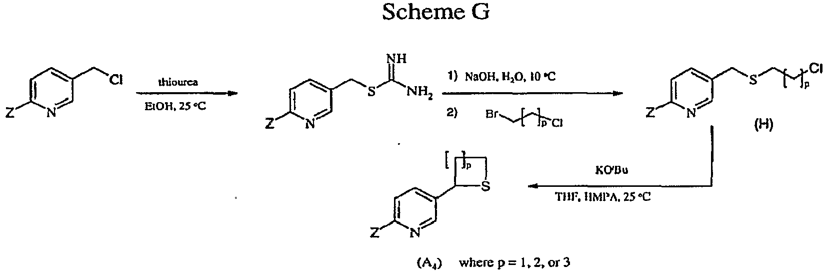

Certain sulfides of formula (A₁) wherein Het is a substituted pyridin-3-yl, Z is as previously defined, and R¹, R² = CH₃ can be prepared alternatively via methods illustrated in Scheme H. Accordingly, the appropriate enone is coupled with dimethylaminoacrylonitrile and cyclized with ammonium acetate in DMF to yield the corresponding 6-substituted nicotinonitrile. Treatment with methylmagnesium bromide, reduction with sodium borohydride, chlorination with thionyl chloride, and nucleophilic substitution with the sodium salt of an alkyl thiol provides desired sulfides (A₁).

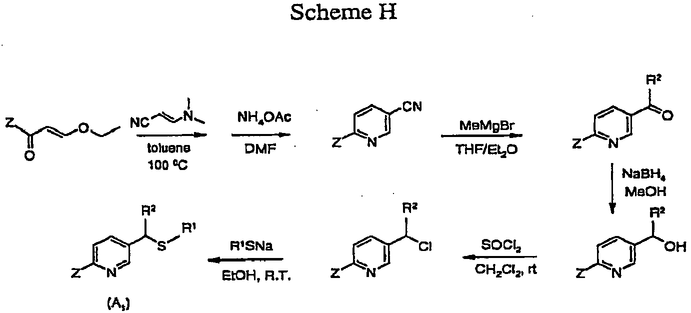

A variation of Scheme H is illustrated in Scheme I, wherein enamines, formed from the addition of an amine, e.g., pyrrolidine, with the Michael adduct of certain sulfides with appropriately substituted α,β-unsaturated aldehydes, are coupled with substituted enones and cyclized with ammonium acetate in CH₃CN to yield the desired sulfides (A₁) wherein R¹, R², R³, and Z are previously defined.

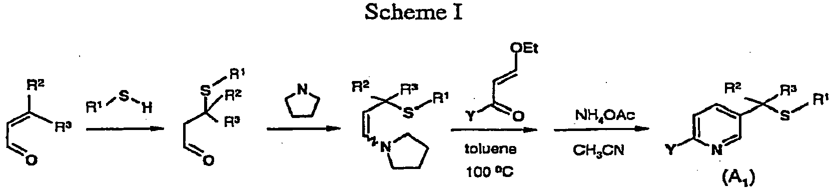

The oxidizing agent employed in the present invention is an alkali metal of permanganate.

Sodium and potassium permanganate are the preferred alkali metal permanganates with sodium permanganate being most preferred. The range of permanganate salt equivalents can be from 0.9 to 1.1 relative to the sulfilimine substrate. The preferred number of equivalents is 0.95. When working up the permanganate reaction mixture it is advisable to quench the excess permanganate. Salts of *meta*-bisulfite (such as sodium or potassium) can be used in the quench step of the workup. The preferred salt of choice is sodium. The number of equivalents of *meta*-bisulfite can range from 1.0 to 5.0 relative to the permanganate stoichiometry. The preferred range of equivalents is from 2.0 to 4.0.

The process of the present invention is conducted in a suitable organic solvent that is essentially inert to the strong oxidizing conditions. Particularly suitable organic solvents are halogenated aliphatic and halogenated aromatic hydrocarbons such as dichloromethane, chloroform, 1,2-dichloroethane and dichlorobenzene, and aliphatic and aromatic nitriles such as acetonitrile and benzonitrile. The preferred reaction solvents are methylene chloride and acetonitrile. It is often convenient to perform the oxidation in a biphasic solvent system comprising a mixture of, for example, a halogenated aliphatic hydrocarbon such as dichloromethane and water.

The reaction temperature can range from -10°C to 45°C. The preferred range is 10°C to 30°C.

The sulfilimine substrate can be dissolved in the organic solvent and conadded to the aqueous solution of oxidizing agent or the solution of aqueous oxidizing agent can be added to the solution of sulfilimine in the organic solvent. The preferred addition order is con-adding the sulfilimine solution to the aqueous solution of the oxidizing agent.

The following examples are presented to illustrate the invention.

### EXAMPLES

### Example 1 (not according to the invention). Preparation of methyl-5-(2-chloro)pyridine-methyl-N-cyanosulfoximine.

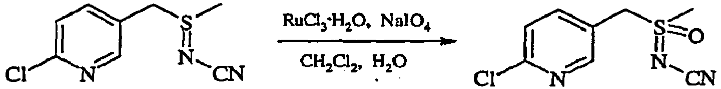

5-(2-chloro)pyridine-methyl-*N*-cyanosulfilimine (151 g, 0.7 mol) was dissolved in 4 liters of dichloromethane and added to a solution of sodium periodate (302 g, 1.4 mol) in 3 liters of water. Ruthenium(III) chloride hydrate (160 mg) was added and the mixture stirred for 20 minutes at room temperature. The organic phase was separated, dried over MgSO₄, treated with charcoal and then filtered and concentrated. The tan solid was triturated in a mixture of acetone and hexane, collected by filtration and dried to 110 g of product. mp 120-122°C. ¹H NMR (300 MHz, CDCl₃) δ 8.5 (d, 1H, *J* = 1.9), 7.9 (dd, 1H, *J =* 1.9, 8.3), 7.6 (d, 1H, *J* = 8.3), 5.1 (s, 2H), 3.45 (s, 3H).

### Example 2 (not according to the invention). Preparation of methyl-5-(2-chloro)pyridine-1-ethyl-N-cyanosulfoximine.

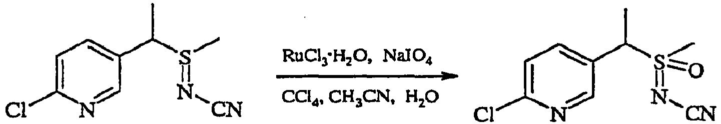

A solution of 300 grams of sodium periodate was prepared in 3.1 liters of water. 2 liters of carbon tetrachloride and 1.7 liters of acetonitrile was added to the solution followed by 1.6 grams of ruthenium(III) chloride hydrate. 5-(2-Chloro)pyridine-1-ethyl-*N*-cyanosulfilimine (161 g, 0.7 mol) was dissolved in 350 milliliters of acetonitrile and added to the stirred mixture at room temperature. After 20 minutes, the organic phase was separated, washed with aqueous NaHSO₃, dried over MgSO₄, treated with charcoal and then filtered and concentrated. The resulting solid was triturated in a mixture of hexane and acetone to give 101 g of a 3:2 mixture of diasteromers as a white solid. mp 102-110°C. ¹H NMR (300 MHz, d₆-DMSO) δ 8.5 (d, 1H), 8.0 (m, 1H), 7.6 (d, 1H), 5.2 (m, 1H), 3.45 (m, 3H); 1.8 (d, 3H).

### Example 3 (not according to the invention). Preparation of methyl-5-(2-chloro-3-nitro)pyridine-methyl-N-cyanosulfoximine.

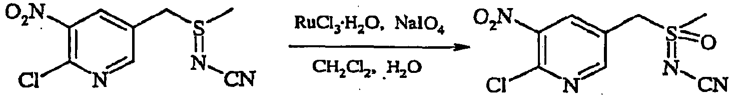

A solution was prepared by adding sodium periodate (661 mg, 3.1 mmol) to 7 milliliters of water at 25°C followed by 7 milliliters of dichloromethane followed by ruthenium(III) chloride hydrate (8.7 mg, 0.04 mmol). 5-(2-Chloro-3-nitro)pyridine-methyl-*N*-cyanosulfilimine (400 mg, 1.5 mmol) was dissolved in 3 milliliters of dichloromethane and added dropwise to the solution at room temperaure. After 20 minutes, the organic phase was separated, dried, filtered and concentrated. The residue was purified by column chromatography to give the product. mp 138-140°C. ¹H NMR (400 MHz, CDCl₃/DMSO) δ 8.44 (d, 1H), 8.31 (d, 1H), 4.82 (s, 2H), 3.04 (s, 3H). LC-MS (ELSD): mass calculated for C₈H₈ClN₄O₃S [M+H]⁺ 275. Found 275.

### Example 4 (not according to the invention). Preparation of methyl-5-(2-chloro-3-methoxy)pyridine-methyl-N-cyanosulfoximine.

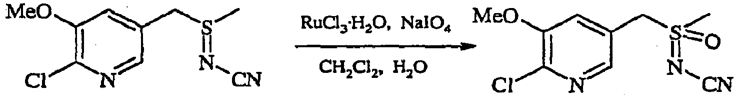

A solution was prepared by adding sodium periodate (351 mg, 1.6 mmol) to 3 milliliters of water at 25°C followed by 3 milliliters of dichloromethane followed by ruthenium(III) chloride hydrate (4.6 mg, 0.021 mmol). 5-(2-Chloro-3-methoxy)pyridine-methyl-*N*-cyanosulfilimine (200 mg, 0.82 mmol) was dissolved in 2.5 milliliters of dichloromethane and added dropwise to the solution and stirred for 30 minutes at room temperature. The organic phase was separated after filtration, dried over Na₂SO₄, filtered and concentrated to a white solid. mp 123-125°C. ¹H NMR (400 MHz, CDCl₃) δ 7.98 (d, 1H), 7.41 (d, 1H), 4.63 (dd, 1H), 3.99 (s, 3H), 3.11 (s, 3H). LC-MS (ELSD): mass calculated for C₉H₁₁ClN₃O₂S [M+H]⁺ 260. Found 260.

### Example 5 (not according to the invention). Preparation of methyl-5-(2-chloro-3-bromo)pyridine-methyl-N-cyanosulfoximine.

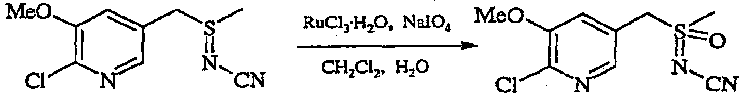

A solution was prepared by adding sodium periodate (246 mg, 1.2 mmol) to 3 milliliters of water at 25°C followed by 3 milliliters of dichloromethane followed by ruthenium(III) chloride hydrate (6.6 mg, 0.029 mmol). 5-(2-Chloro-3-bromo)pyridine-methyl-*N*-cyanosulfilimine (170 mg, 0.6 mmol) was dissolved in 2 milliliters of dichloromethane and added dropwise to the solution and stirred 1 hour at room temperature. The organic phase was separated, dried over MgSO₄, filtered and concentrated to a white solid. mp 139-142°C. ¹H NMR (400 MHz, CDCl₃/DMSO) δ 8.6 (d, 1H), 8.4 (d, 1H), 5.1 (s, 2H), 3.5 (s, 3H). LC-MS (ELSD): mass calculated for C₈H₇BrClN₃OS [M+H]⁺ 308. Found 308.

### Example 6 (not according to the invention). Preparation of methyl-5-(2-methoxy)pyridine-methyl-N-cyanosulfoximine.

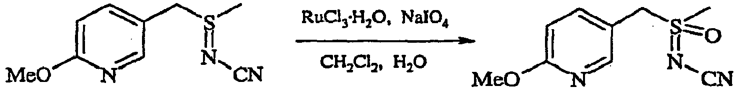

A solution was prepared by adding sodium periodate (818 mg, 3.8 mmol) to 6 milliliters of water at 25°C followed by 6 milliliters of dichloromethane followed by Ruthenium(III) chloride hydrate (22 mg, 0.095 mmol). 5-(2-methoxy)pyridine-methyl-*N*-cyanosulfilimine (400 mg, 1.9 mmol) was dissolved in 3 milliliters of dichloromethane and added dropwise to the solution. The reaction was diluted with CH₂Cl₂ (10 milliliters) and passed through a diatomaceous earth plug. The organic phase was separated, dried over MgSO₄, filtered and concentrated to furnish the sulfoximine as a yellow solid. mp = 89-91°C. ¹H NMR (400 MHz, CDCl₃/DMSO) δ 8.2 (d, 1H), 7.7 (dd, 1H), 6.9 (d, 1H), 4.5 (s, 2H), 4.0 (s, 3H), 3.1 (s, 3H). LC-MS (ELSD): mass calculated for C₉H₁₁N₃O₂S [M+H]⁺ 225. Found 225.

### Example 7 (not according to the invention). Preparation of 3-[5-(2-trifluoromethyl)pyridine-methyl-N-cyano-cyclo-pentylsulfoximine.

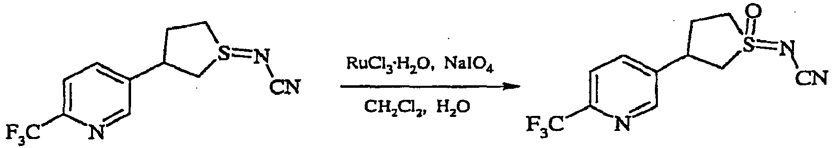

A solution was prepared by adding sodium periodate (861 mg, 4.07 mmol) to 14 milliliters of water followed by 24 milliliters of dichloromethane followed by ruthenium(III) chloride hydrate (8 mg, 0.04 mmol). 3-[5-(2-Trifluoromethyl)-pyridine-*N*-cyano-cyclopentylsulfilimine (1.00 mg, 3.66 mmol) was added to the solution. The solution was stirred overnight at room temperature. Isopropyl alcohol (0.5 milliliters) was added to the solution. The reaction was passed through a diatomaceous earth pad. The organic phase was separated, dried over MgSO₄, filtered and concentrated to furnish the sulfoximine as a off-white solid (360 mg, 34%). ¹H NMR (400 MHz, acetone-d₆) δ 8.89 (overlapping doublets, 1H), 8.25 (m, 1H), 7.9 (overlapping doublets, 1H), 4.4 - 3.9 (m, 2H), 3.8 - 3.6 (m, 3H), 3.0 - 2.5 (m, 2H).

### Example 8. Preparation of methyl-5-(2-trifluoromethyl)pyridine-1-ethyl-N-cyanosulfoximine.

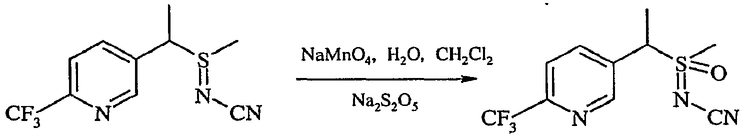

To a four neck 5 L round bottom flask fitted with an addition funnel, reflux condenser, mechanical stirring, and thermowell was charged with 1472 g (0.845 mol) of a 15% w/w of sulfilimine in dichloromethane. The solution was cooled to 3°C in an ice-water bath with stirring. To this solution was added 299 g (0.845 mol) of a 40% w/w sodium permanganate aqueous solution dropwise via addition funnel over a 2 h period. The addition rate was controlled so that the internal temperature rose from 3°C to 11°C during the permanganate addition. The addition funnel was rinsed with 80 mL of water. The reaction was then allowed to stir with ice bath cooling for 1 h. To this mixture was added a solution of 645 g of sodium metabisulfite (3.38 mol) in 1200 mL of water over a 1.5 h period. A definite exotherm was noted during the initial addition of the bisulfite solution (internal solution temp rose from 3°C to 30°C). An additional 250 mL of water was added and the reaction was allowed to stir an additional 2 h until all of brown manganese by-products were etched away from the reactor vessel walls. To this mixture was added 180 mL of acetonitrile. About 2 L of the reaction mixture was suction filtered through a coarse glass fritted funnel (filtration was fast), and the filter cake was washed with 250 mL of dichloromethane. The organic layer was then concentrated on a rotovap. The remaining portion of the reaction mixture was filtered through the same fritted funnel and the filter cake was washed with another 250 mL of dichloromethane. The bottom organic layer was collected and added to the other portion and concentrated on a rotovap to give 228 g (97% yield based on theortical) of an off-white solid. LC assay of this crude material indicated that the purity was 96%:

### Example 9. Preparation of methyl-5-(2-trifluoromethyl)pyridine-1-ethyl-N-cyanosulfoximine.

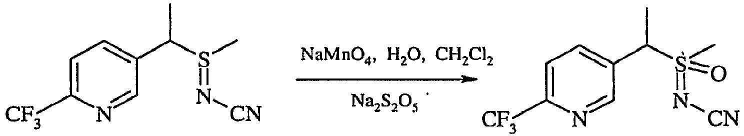

In a 5 L 4-neck round bottom flask, a mixture of 400 mL of dichloromethane, 400 mL of water, and 320 mL (1.25 mol) of a 40% aq solution of NaMnO₄ was cooled to 13°C with an ice- bath. To this rapidly stirred mixture was added dropwise a solution of (∼1.0 mol) sulfilimine in 1000 mL of dichloromethane (∼1560 g) over 1 3/4 h. During this time the ice-bath was lowered or raised to maintain a reaction temperature of 13-20°C. After stirring for 30 min at 15°C, a solution of 570 g (3.0 mol, 3 equiv) of sodium metabisulfite in 900 mL of water was added with rapid stirring over 1.5 h. Very exothermic, the temperature rose from 15-28°C rapidly at first. The mixture was stirred at RT (23°C) for 30 min, and then filtered. The solid was rinsed with two wet cake volumes of dichloromethane. The clear two phase mixture was transferred to a 4 L separatory funnel, and the bottom organics collected. The aqueous layer was reextracted with 30 mL of dichloromethane, and the organics combined with the first cut. The solution was concentrated *in vacuo* to give 275 g of a white solid. This solid was air-dried overnight in a hood to give 260 g and finally in a vacuum oven at 40°C to give 259 g (93% wt) of a white solid. LC analysis indicated a 30:68 (area) ratio of two isomers and a 97% area purity.

### Example 10. Preparation of methyl-5-(2-trifluoromethyl)pyridine-1-ethyl-N-cyanosulfoximine.

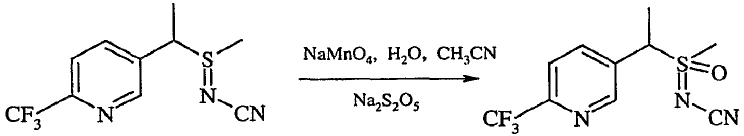

A solution of sulfilimine (∼ 0.022 moles) in acetonitrile (50 mL) was cooled in an ice bath to 5°C. To the well stirred solution was added (8.0 grams, 0.022 moles) of a 40 weight% aqueous solution of NaMnO₄ over 20 minutes. During the addition the reaction temperature increased to 24°C. The resulting brown reaction slurry was allowed to stir for 30 minutes and then cooled to 5°C. A 30 weight% aqueous solution of sodium metabisulfite (29.8 grams, 0.047 moles) was added to the vigorously stirred reaction mixture in portions during 20 minutes. The addition is exothermic, the temperature increasing by 15 to 20°C during the course of the addition. The reaction mixture slurry thickened during the addition. Additional acetonitrile (5 mL) and water (5 mL) were added to facilitate mixing. The quenched reaction mixture was vacuum filtered through a medium sintered glass filter funnel. The collected grey solids were rinsed with acetonitrile (5 mL). The combined filtrate and wash was transferred to a separatory funnel, the phases were allowed to separate and the lower aqueous phase removed. The upper organic phase was concentrated *in vacuo,* with an isopropyl alcohol solvent chase (40 grams) to afford 5.2 grams (83% weight recovery) of crude sulfoximine as a yellow solid. Recrystallization from isopropyl alcohol (4 mL) gave 3.3 grams (52%) of sulfoximine as a white solid. LC analysis indicated a 81:19 (area) ratio of the two isomers and a 89% area purity.

## Claims

1. A process for the preparation of insecticidal sulfoximines (Ia), 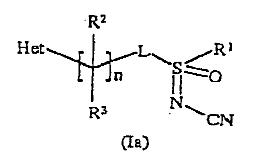 wherein
Het represents: 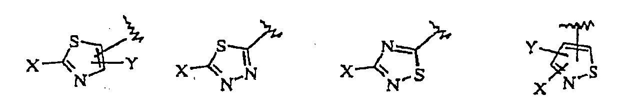 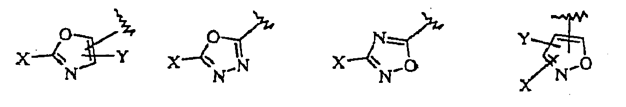 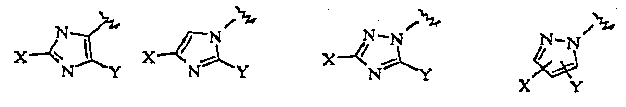 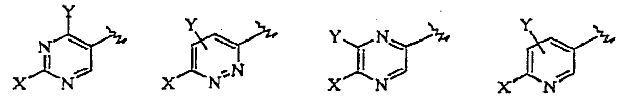
X represents halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, NO₂, SOₘR⁶ where m is an integer from 0-2, COOR⁴ or CONR⁴R⁵;
Y represents hydrogen, halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, NO₂, SOₘR¹ where m is an integer from 0-2, COOR⁴, CONR⁴R⁵, aryl or heteroaryl;
n is an integer from 0-3;
L represents either a single bond, -CH(CH₂)ₚ- where R¹, S and L taken together represent a 4-, 5-, or 6-membered ring and p is an integer from 1-3, -CH(CH₂OCH₂)-where R¹, S and L taken together represent a 6-membered ring, or -CH- where L, R² and the common carbon to which they connect taken together represent a 4-, 5-, or 6-membered ring with up to, but no more than, 1 heteratom.
R¹ represents C₁-C₄ alkyl, C₁-C₄ haloalkyl, C₃-C₆ alkenyl, C₃-C₆ alkynyl, C₃-C₆ haloalkenyl, arylalkyl, heteroarylalkyl, or -CH₂- in cases where R¹, S and L taken together represent a 4-, 5-, or 6-membered ring;
R² and R³ independently represent hydrogen, halogen, C₁-C₄ alkyl, C₁-C₄ haloalkyl, C₂-C₄ alkenyl, C₂-C₄ alkynyl, C₂-C₄ haloalkenyl, C₁-C₄ alkoxy, C₁-C₄ haloalkoxy, CN, SOₘR⁶ where m is an integer from 0-2, COOR⁴, CONR⁴R⁵, arylalkyl, heteroarylalkyl, or R² and R³ and the common carbon to which they attach form a 3-6 membered ring;
R⁴ and R⁵ independently represent hydrogen, C₁-C₄ alkyl, C₁-C₄ haloalkyl; C₃-C₆ alkenyl, C₃-C₆ alkynyl, C₃-C₆ haloalkenyl, aryl, heteroaryl, arylalkyl or heteroarylalkyl; and
R⁶ represents C₁-C₄ alkyl, C₁-C₄ haloalkyl, C₃-C₆ alkenyl, C₃-C₆ alkynyl, C₃-C₆ haloalkenyl, arylalkyl or heteroarylalkyl; and
wherein "alkyl", "alkenyl", "alkynyl" and "alkoxy" include straight chain, branched chain and cyclic moieties, and each may be unsubstituted or substituted with one or more substituents selected from but not limited to halogen, hydroxy, alkoxy, alkylthio, C₁-C₆ acyl, formyl, cyano, aryloxy or aryl, provided that the substituents are sterically compatible and the rules of chemical bonding and strain energy are satisfied; and "aryl" refers to a phenyl, indanyl or naphthyl group; and "aryl" and "heteroaryl" may be unsubstituted or substituted with one or more substituents selected from halogen, hydroxy, nitro, cyano, aryloxy, formyl C₁-C₆ alkyl, C₂-C₆ alkenyl, C₂-C₆ alkynyl, C₁-C₆ alkoxy, halogenated C₁-C₆ alkyl, haolgenated C₁-C₆ alkoxy, C₁-C₆ acyl, C₁-C₆ alkylthio, C₁-C₆ alkylsulfinyl, C₁-C₆ alkylsulfonyl, aryl, C₁-C₆OC(O)alkyl, C₁-C₆NHC(O)alkyl, C(O)OH, C₁-C₆C(O)Oalkyl, C(O)NH₂, C₁-C₆C(O)NHalkyl, or C₁-C₆ C(O)N(alkyl)₂, provided that the substituents are sterically compatible and the rules of chemical bonding and strain energy are satisfied;
which comprises oxidizing a sulfilimine of formula (I) 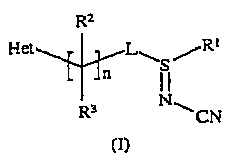 wherein
R¹, R², R³, L, Het and n are as previously defined by contacting the sulfilimine in a suitable organic solvent that is essentially inert to the strong oxidizing conditions with an oxidizing agent comprising an alkali metal permanganate at a temperature from -10 to 45°C.

2. The process of Claim 1 in which Het is (6-substituted)pyridin-3-yl or (2-substituted)thiazol-5-yl and where X is halogen or C₁-C₂ haloalkyl and Y is hydrogen.

3. The process of Claim 1 in which the starting sulfilimine has the structure 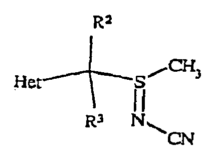 wherein
Het, R² and R³ are as previously defined.

4. The process of Claim 1 in which the starting sulfilimine has the structure 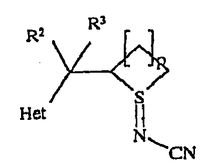 wherein
p is an integer from 1-3, and Het, R² and R³ are as previously defined.

5. The process of Claim 1 in which the starting sulfilimine has the structure 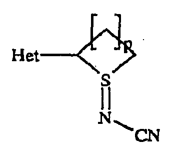 wherein p is an integer from 1-3, and Het is as previously defined.

6. The process of Claim 1 in which the temperature is from 10°C to 30°C.

7. The process of Claim 1 in which the organic solvent is a halogenated aliphatic or halogenated aromatic hydrocarbon or an aliphatic or aromatic nitrile.

8. The process of Claim 1 in which the process is conducted in a biphasic solvent system comprising a mixture of a halogenated aliphatic hydrocarbon and water.

## Patentansprüche

1. Verfahren zur Herstellung von insektiziden Sulfoximinen (la) 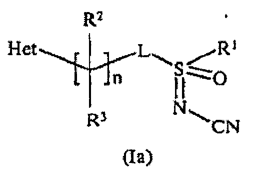 wobei
Het 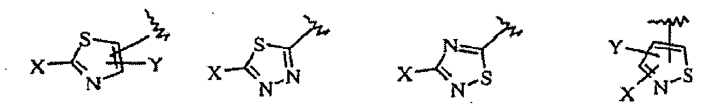 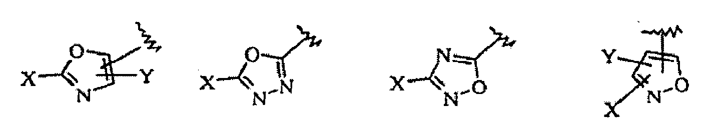 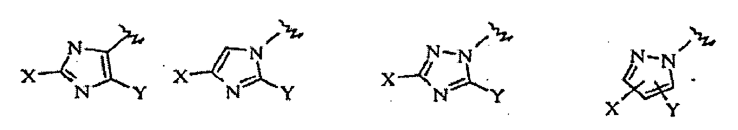 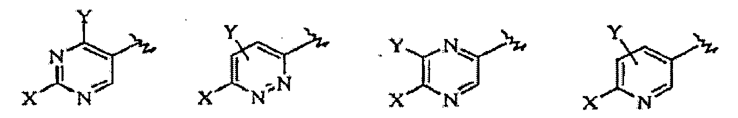 darstellt;
X Halogen, C₁-C₄-Alkyl, C₁-C₄-Haloalkyl, C₂-C₄-Alkenyl, C₂-C₄-Alkinyl, C₂-C₄-Haloalkenyl, C₁-C₄-Alkoxy, C₁-C₄-Haloalkoxy, CN, NO₂, SOₘR⁶, wobei m eine ganze Zahl von 0-2 ist, COOR⁴ oder CONR⁴R⁵ darstellt;
Y Wasserstoff, Halogen, C₁-C₄-Alkyl, C₁-C₄-Haloalkyl, C₂-C₄-Alkenyl, C₂-C₄-Alkinyl, C₂-C₄-Haloalkenyl, C₁-C₄-Alkoxy, C₁-C₄-Haloalkoxy, CN, NO₂, SOₘR¹, wobei m eine ganze Zahl von 0-2 ist, COOR⁴ oder CONR⁴R⁵, Aryl oder Heteroaryl darstellt;
n eine ganze Zahl von 0-3 ist;
L entweder eine einfache Bindung, -CH(CH₂)ₚ-, wobei R¹, S und L zusammengenommen einen 4-, 5- oder 6-gliedrigen Ring darstellen und p eine ganze Zahl von 1-3 ist, -CH(CH₂OCH₂)-, wobei R¹, S und L zusammengenommen einen 6-gliedrigen Ring darstellen oder -CH-, wobei L, R² und der gemeinsame Kohlenstoff an den sie binden zusammengenommen einen 4-, 5- oder 6-gliedrigen Ring mit bis zu, aber nicht mehr als 1 Heteroatom darstellen, darstellt;
R¹ C₁-C₄-Alkyl, C₁-C₄-Haloalkyl, C₃-C₆-Alkenyl, C₃-C₆-Alkinyl, C₃-C₆-Haloalkenyl, Arylalkyl, Heteroarylalkyl oder -CH₂- in den Fällen, in denen R¹, S und L zusammengenommen einen 4-, 5- oder 6-gliedrigen Ring darstellen, darstellt;
R² und R³ unabhängig voneinander Wasserstoff, Halogen, C₁-C₄-Alkyl, C₁-C₄-Haloalkyl, C₂-C₄-Alkenyl, C₂-C₄-Alkinyl, C₂-C₄-Haloalkenyl, C₁-C₄-Alkoxy, C₁-C₄-Haloalkoxy, CN, SOₘR⁶, wobei m eine ganze Zahl von 0-2 ist, COOR⁴, CONR⁴R⁵, Arylalkyl, Heteroarylalkyl oder R² und R³ und der gemeinsame Kohlenstoff an den sie binden zusammengenommen einen 3-6-gliedrigen Ring bilden, darstellen;
R⁴ und R⁵ unabhängig voneinander Wasserstoff, C₁-C₄-Alkyl, C₁-C₄-Haloalkyl, C₃-C₆-Alkenyl, C₃-C₆-Alkinyl, C₃-C₆-Haloalkenyl, Aryl, Heteroaryl, Arylalkyl oder Heteroarylalkyl darstellen; und
R⁶ C₁-C₄-Alkyl, C₁-C₄-Haloalkyl, C₃-C₆-Alkenyl, C₃-C₆-Alkinyl, C₃-C₆-Haloalkenyl, Arylalkyl oder Heteroarylalkyl darstellt; und
wobei "Alkyl", "Alkenyl", "Alkinyl" und "Alkoxy" geradkettige, verzweigtkettige und zyklische Reste beinhalten, und jeder unsubstituiert oder mit einem oder mehreren Substituenten ausgewählt aus, aber nicht beschränkt auf Halogen, Hydroxy, Alkoxy, Alkylthio, C₁-C₆-Acyl, Formyl, Cyano, Aryloxy oder Aryl substituiert sein kann, vorausgesetzt, dass die Substituenten sterisch kompatibel sind und die Regeln der chemischen Bindung und Spannungsenergie eingehalten werden; und
"Aryl" sich auf eine Phenyl-, Indanyl- oder Naphtylgruppe bezieht; und "Aryl" und "Heteroaryl" unsubstituiert oder mit einem oder mehreren Substituenten ausgewählt aus Halogen, Hydroxy, Nitro, Cyano, Aryloxy, Formyl, C₁-C₆-Alkyl, C₂-C₆-Alkenyl, C₂-C₆-Alkinyl, C₁-C₆-Alkoxy, halogeniertem C₁-C₆-Alkyl, halogeniertem C₁-C₆-Alkoxy, C₁-C₆-Acyl, C₁-C₆-Alkylthio, C₁-C₆-Alkylsulfinyl, C₁-C₆-Alkylsulfonyl, Aryl, C₁-C₆OC(O)Alkyl, C₁-C₆NHC(O)Alkyl, C(O)OH, C₁-C₆C(O)OAlkyl, C(O)NH₂, C₁-C₆C(O)NHAlkyl oder C₁-C₆C(O)N(Alkyl)₂ substituiert sein können, vorausgesetzt, dass die Substituenten sterisch kompatibel sind und die Regeln der chemischen Bindung und Spannungsenergie eingehalten werden;
welches Oxidieren eines Sulfilimins der Formel (I) 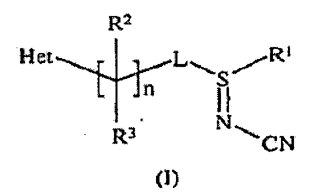 wobei
R¹, R², R³, L, Het und n wie vorhergehend definiert sind
durch Inkontaktbringen des Sulfilimins in einem geeigneten organischen Lösungsmittel, das im Wesentlichen gegenüber starken Oxidationsbedingungen inert ist, mit einem Oxidationsmittel umfassend ein Alkalimetallpermanganat bei einer Temperatur von -10 bis 45°C umfasst.

2. Verfahren nach Anspruch 1, bei dem Het (6-substituiertes)Pyridin-3-yl oder (2-substituiertes)Thiazol-5-yl ist und wobei X Halogen oder C₁-C₂-Haloalkyl ist und Y Wasserstoff ist.

3. Verfahren nach Anspruch 1, bei dem das Ausgangs-Sulfilimin die Struktur 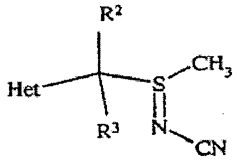 aufweist,
wobei Het, R² und R³ wie vorhergehend definiert sind.

4. Verfahren nach Anspruch 1, bei dem das Ausgangs-Sulfilimin die Struktur 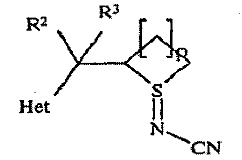 aufweist,
wobei p eine ganze Zahl von 1-3 ist und Het, R² und R³ wie vorhergehend definiert sind.

5. Verfahren nach Anspruch 1, bei dem das Ausgangs-Sulfilimin die Struktur 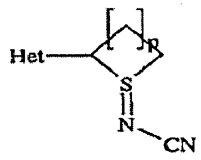 aufweist,
wobei p eine ganze Zahl von 1-3 ist und Het wie vorhergehend definiert ist.

6. Verfahren nach Anspruch 1, bei dem die Temperatur von 10°C bis 30°C ist.

7. Verfahren nach Anspruch 1, bei dem das organische Lösungsmittel ein halogenierter aliphatischer oder halogenierter aromatischer Kohlenwasserstoff oder ein aliphatisches oder aromatisches Nitril ist.

8. Verfahren nach Anspruch 1, bei dem das Verfahren in einem zweiphasigen Lösungsmittelsystem umfassend eine Mischung aus einem halogenierten aliphatischen Kohlenwasserstoff und Wasser durchgeführt wird.

## Revendications

1. Procédé pour la préparation de sulfoximines insecticides (Ia), 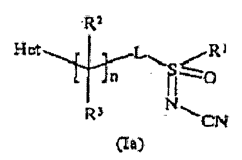 où
Het représente : 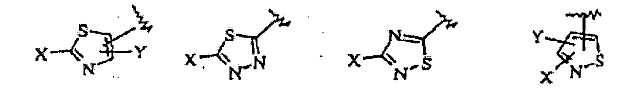 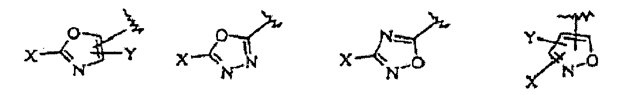 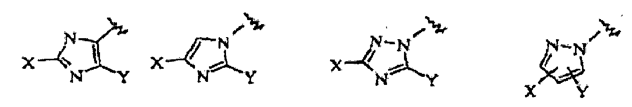 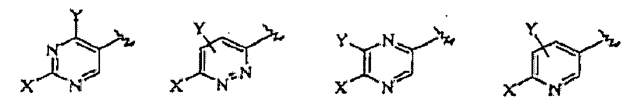
X représente un atome d'halogène, un groupe alkyle en C₁-C₄, halogénoalkyle(C₁-C₄), alcényle en C₂-C₄, alcynyle en C₂-C₄, halogéno-alcényle(C₂-C₄), alcoxy en C₁-C₄, halogénoalcoxy(C₁-C₄), CN, NO₂, SOₘR⁶, m étant un nombre entier valant de 0 à 2, COOR⁴ ou CONR⁴R⁵,
Y représente un atome d'hydrogène ou d'halogène, un groupe alkyle en C₁-C₄, halogénoalkyle(C₁-C₄), alcényle en C₂-C₄, alcynyle en C₂-C₄, halogénoalcényle(C₂-C₄), alcoxy en C₁-C₄, halogénoalcoxy(C₁-C₄), CN, NO₂, SOₘR¹, m étant un nombre entier valant de 0 à 2, COOR⁴, CONR⁴R⁵, aryle ou hétéroaryle ;
n est un nombre entier valant de 0 à 3 ;
L représente soit une simple liaison, soit un groupe -CH(CH₂)ₚ-, R¹, S et L pris ensemble représentant un cycle à 4, 5 ou 6 chaînons et p étant un nombre entier valant de 1 à 3, -CH(CH₂OCH₂)-, R¹, S et L pris ensemble représentant un cycle à 6 chaînons, ou -CH-, L , R² et l'atome de carbone commun auquel ils sont liés, pris ensemble, représentent un cycle à 4, 5 ou 6 chaînons, comportant jusqu'à, mais au maximum, 1 hétéroatome,
R¹ représente un groupe alkyle en C₁-C₄, halogénoalkyle(C₁-C₄), alcényle en C₃-C₆, alcynyle en C₃-C₆, halogénoalcényle(C₃-C₆), arylalkyle, hétéroarylalkyle, ou -CH₂- dans les cas où R¹, S et L pris ensemble représentent un cycle à 4, 5 ou 6 chaînons ;
R² et R³ représentent indépendamment un atome d'hydrogène ou d'halogène, un groupe alkyle en C₁-C₄, halogénoalkyle(C₁-C₄), alcényle en C₂-C₄, alcynyle en C₂-C₄, halogénoalcényle(C₂-C₄), alcoxy en C₁-C₄, halogénoalcoxy(C₁-C₄), CN, SOₘR⁶, m étant un nombre entier valant de 0 à 2, COOR⁴, CONR⁴R⁵, arylalkyle, hétéroarylalkyle, ou R² et R³ et l'atome de carbone commun auquel ils sont liés forment un cycle à 3-6 chaînons ;
R⁴ et R⁵ représentent indépendamment un atome d'hydrogène, un groupe alkyle en C₁-C₄, halogénoalkyle(C₁-C₄) ; alcényle en C₃-C₆, alcynyle en C₃-C₆, halogénoalcényle(C₃-C₆), aryle, hétéroaryle, arylalkyle ou hétéroarylalkyle ; et
R⁶ représente un groupe alkyle en C₁-C₄, halogénoalkyle(C₁-C₄), alcényle en C₃-C₆, alcynyle en C₃-C₆, halogénoalcényle(C₃-C₆), arylalkyle ou hétéroarylalkyle ; et
les groupes « alkyle », « alcényle », « alcynyle » et « alcoxy » incluant des groupes à chaîne droite, à chaîne ramifiée et cycliques, et chacun pouvant être non substitué ou porter un ou plusieurs substituant(s) choisi(s), mais sana limitation, parmi des atomes d'halogène, des groupes hydroxyle, alcoxy, alkylthio, acyle en C₁-C₆, formyle, cyano, aryloxy ou aryle, à la condition que les substituants soient stériquement compatibles et que les règles de la liaison chimique et de l'énergie de déformation soient satisfaites ; et « aryle » signifie un groupe phényle, indanyle ou naphtyle ; et les groupes « aryle » et « hétéroaryle » peuvent être non substitués ou porter un ou plusieurs substituant(s) choisi(s) parmi des atomes d'halogène, des groupes hydroxyle, nitro, cyano, aryloxy, formyle, alkyle en C₁-C₆, alcényle en C₂-C₆, alcynyle en C₂-C₆, alcoxy en C₁-C₆, alkyle en C₁-C₆ halogéné, alcoxy en C₁-C₆ halogéné, acyle en C₁-C₆, alkyl(C₁-C₆)thio, alkyl(C₁-C₆)sulfinyle, alkyl(C₁-C₆)sulfonyle, aryle, OC(O)alkyle(C₁-C₆), NHC(O)alkyle(C₁-C₆), C(O)OH, C(O)Oalkyle(C₁-C₆), C(O)NH₂, C(O)NHalkyle(C₁-C₆) ou C(O)N(alkyle(C₁-C₆))₂, à la condition que les substituants soient stériquement compatibles et que les règles de la liaison chimique et de l'énergie de déformation soient satisfaites ;
qui comprend l'oxydation d'une sulfilimine de formule (I) 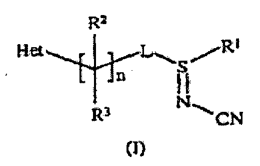 dans laquelle
R¹, R², R³, L, Het et n sont tels que définis précédemment
par mise en contact de la sulfilimine, dans un solvant organique convenable qui est essentiellement inerte vis-à-vis des conditions de forte oxydation, avec un agent oxydant comprenant un permanganate de métal alcalin, à une température de -10 à 45 °C.

2. Procédé selon la revendication 1, dans lequel Het est le groupe pyridin-3-yle substitué en position 6 ou thiazol-5-yle substitué en position 2 et dans lequel X est un atome d'halogène ou un groupe halogénoalkyle(C₁-C₂) et Y est un atome d'hydrogène.

3. Procédé selon la revendication 1, dans lequel la sulfilimine de départ a la structure 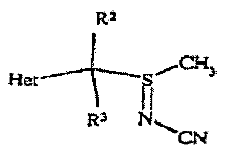 dans laquelle
Het, R² et R³ sont tels que définis précédemment.

4. Procédé selon la revendication 1, dans lequel la sulfilimine de départ a la structure 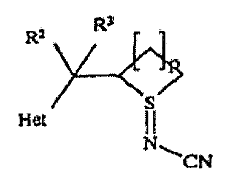 dans laquelle
p est un nombre entier valant de 1 à 3, et Het, R² et R³ sont tels que définis précédemment.

5. Procédé selon la revendication 1, dans lequel la sulfilimine de départ a la structure 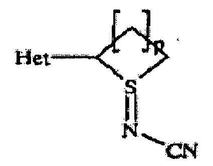 dans laquelle
p est un nombre entier valant de 1 à 3, et Het est tel que défini précédemment.

6. Procédé selon la revendication 1, dans lequel la température est dans la plage de 10 °C à 30 °C.

7. Procédé selon la revendication 1, dans lequel le solvant organique est un hydrocarbure aliphatique halogéné ou aromatique halogéné ou un nitrile aliphatique ou aromatique.

8. Procédé selon la revendication 1, dans lequel le procédé est mis en oeuvre dans un système solvant en deux phases, comprenant un mélange d'un hydrocarbure aliphatique halogéné et de l'eau.
